# EUROPEAN PATENT APPLICATION

(11) **EP 4 793 268 A1**
(43) Date of publication of application: **19.08.2026**
(21) Application number: 24877145.3
(22) Date of filing: 07.10.2024
(51) Int. Cl.: C07C 265/14, C07C 263/10, C07C 263/18, C07C 321/14, C08G 18/38, C08G 18/76, G02B 1/04

(54) **XYLYLENE DIISOCYANATE COMPOSITION, POLYMERIZABLE COMPOSITION, RESIN, MOLDED BODY, OPTICAL ELEMENT, AND LENS**

(30) Priority: 12.10.2023 JP 2023177121
(71) Applicant: Mitsui Chemicals, Inc., Tokyo 104-0028 (JP)
(72) Inventor: NAKAI, Shinnosuke, Omuta-shi, Fukuoka 836-8610 (JP)
(74) Representative: Mewburn Ellis LLP
(86) International application number: PCT/JP2024/035768
(87) International publication number: WO 2025/079550

(57) **Abstract**

A xylylene diisocyanate composition includes a xylylene diisocyanate and isocyanatomethylbenzoyl chloride.

## Description

### TECHNICAL FIELD

The present invention relates to a xylylene diisocyanate composition, a polymerizable composition, a resin, a molded article, an optical element, and a lens.

### BACKGROUND ART

Conventionally, it has been known to react a xylylene diisocyanate composition with a polythiol to produce a resin which can be used for an optical element such as a lens (see Patent Document 1 below).

### Citation List

### Patent Document

Patent Document 1: International Publication No. WO2018/190290

### SUMMARY OF THE INVENTION

### PROBLEM TO BE SOLVED BY THE INVENTION

In the production of a resin as described in Patent Document 1, further improvement in heat resistance and also the suppression of decrease in light resistance may be required.

The present invention provides a xylylene diisocyanate composition and a polymerizable composition which allow for the production of a resin having excellent light resistance and heat resistance, and a resin, a molded article, an optical element, and a lens which have excellent light resistance and heat resistance.

### MEANS FOR SOLVING THE PROBLEM

The present invention [1] includes a xylylene diisocyanate composition comprising: a xylylene diisocyanate; and isocyanatomethylbenzoyl chloride.

The present invention [2] includes the xylylene diisocyanate composition described in the above-described [1], wherein a ratio of the isocyanatomethylbenzoyl chloride in the xylylene diisocyanate composition is 1 ppm or more based on mass.

The present invention [3] includes the xylylene diisocyanate composition described in the above-described [1] or [2], wherein a ratio of the isocyanatomethylbenzoyl chloride in the xylylene diisocyanate composition is 2000 ppm or less based on mass.

The present invention [4] includes the xylylene diisocyanate composition described in any one of the above-described [1] to [3], further containing isocyanatomethylbenzoic acid.

The present invention [5] includes a polymerizable composition including: the xylylene diisocyanate composition described in any one of the above-described [1] to [4]; and an active hydrogen group-containing component.

The present invention [6] includes the polymerizable composition described in the above-described [5], wherein the active hydrogen group-containing component contains at least one type of polythiol selected from the group consisting of 5,7-dimercaptomethyl-1,11-dimercapto-3,6,9-trithiaundecan, 4,7-dimercaptomethyl-1,11-dimercapto-3,6,9-trithiaundecan, 4,8-dimercaptomethyl-1,11-dimercapto-3,6,9-trithiaundecan, 4-mercaptomethyl-1,8-dimercapto-3,6-dithiaoctane, pentaerythritol tetrakis(2-mercaptoacetate), pentaerythritol tetrakis(3-mercaptopropionate), 2,5-bis(mercaptomethyl)-1,4-dithiane, bis(mercaptoethyl)sulfide, 1,1,3,3-tetrakis(mercaptomethylthio)propane, 4,6-bis(mercaptomethylthio)-1,3-dithiane, 2-(2,2-bis(mercaptomethylthio)ethyl)-1,3-dithiethane, 1,1,2,2-tetrakis(mercaptomethylthio)ethane, 3-mercaptomethyl-1,5-dimercapto-2,4-dithiapentane, tris(mercaptomethylthio)methane, and ethylene glycol bis(3-mercaptopropionate).

The present invention [7] includes a resin being a cured product of the polymerizable composition described in the above-described [5] or [6].

The present invention [8] includes a molded article made of the resin described in the above-described [7].

The present invention [9] includes an optical element being the molded article described in the above-described [8].

The present invention [10] includes a lens being the optical element described in the above-described [9].

### EFFECTS OF THE INVENTION

The xylylene diisocyanate composition and polymerizable composition of the present invention contain isocyanatomethylbenzoyl chloride.

By using the xylylene diisocyanate composition and polymerizable composition that contain isocyanatomethylbenzoyl chloride as a raw material, a resin having excellent light resistance and heat resistance can be produced.

The resin of the present invention is a cured product of the polymerizable composition containing the above-described xylylene diisocyanate composition.

Further, the molded article, optical element, and lens of the present invention are made of the above-described resin.

Therefore, the resin, molded article, optical element, and lens of the present invention have excellent light resistance and heat resistance.

### DESCRIPTION OF THE EMBODIMENTS

### 1. Xylylene Diisocyanate Composition

The xylylene diisocyanate composition (XDI composition) contains a xylylene diisocyanate (XDI) as a primary component. The "XDI composition" described here is a "second XDI composition" obtained by a "method of producing a XDI composition" described later.

Examples of the XDI include 1,2-XDI (o-XDI), 1,3-XDI (m-XDI), and 1,4-XDI (p-XDI).

As the XDI, preferably, 1,3-XDI(m-XDI) is used.

The XDI composition may contain two or more types of XDI.

The ratio (purity) of XDI in the XDI composition is, for example, 98.00% by mass or more, preferably 99.00% by mass or more, more preferably 99.30% by mass or more, or 99.60% by mass or more.

The ratio of XDI in the XDI composition is, for example, 99.95% by mass or less.

The above-described upper and lower limits of the ratio of XDI in the XDI composition can be combined to set a range of the ratio of XDI in the XDI composition. The ratio of XDI in the XDI composition may be 98.00% by mass to 99.95% by mass, 99.00% by mass to 99.95% by mass, 99.30% by mass to 99.95% by mass, or 99.60% by mass to 99.95% by mass.

The ratio of XDI in the XDI composition is measured by the method described in Examples described later.

The XDI composition contains isocyanatomethylbenzoyl chloride as a secondary component.

Examples of the isocyanatomethylbenzoyl chloride include 2-isocyanatomethylbenzoyl chloride (o-isocyanatomethylbenzoyl chloride), 3-isocyanatomethylbenzoyl chloride (m-isocyanatomethylbenzoyl chloride), and 4-isocyanatomethylbenzoyl chloride (p-isocyanatomethylbenzoyl chloride).

As the isocyanatomethylbenzoyl chloride, 3-isocyanatomethylbenzoyl chloride (m-isocyanatomethylbenzoyl chloride) is preferably used.

The XDI composition may contain two or more types of isocyanatomethylbenzoyl chloride.

The ratio of the isocyanatomethylbenzoyl chloride in the XDI composition is, based on mass, for example, 1 ppm or more, 3 ppm or more, 5 ppm or more, 7 ppm or more, 10 ppm or more, 15 ppm or more, 20 ppm or more, 25 ppm or more, 30 ppm or more, 35 ppm or more, 40 ppm or more, 45 ppm or more, and 50 ppm or more.

When the ratio of the isocyanatomethylbenzoyl chloride in the XDI composition is the above-described lower limit or more, the heat resistance of the resin produced using the XDI composition can be improved.

The ratio of the isocyanatomethylbenzoyl chloride in the XDI composition is, based on mass, for example, 5000 ppm or less, 2000 ppm or less, 1900 ppm or less, 1500 ppm or less, 1000 ppm or less, 800 ppm or less, 500 ppm or less, 300 ppm or less, 200 ppm or less, 100 ppm or less, 90 ppm or less, and 70 ppm or less.

When the ratio of the isocyanatomethylbenzoyl chloride in the XDI composition is the above-described upper limit or less, the reduction in light resistance of the resin produced using the XDI composition can be suppressed.

The above-described upper and lower limits of the ratio of isocyanatomethylbenzoyl chloride in the XDI composition can be combined to set a range of the ratio of isocyanatomethylbenzoyl chloride in the XDI composition. The ratio of isocyanatomethylbenzoyl chloride in the XDI composition may be 1 ppm to 5000 ppm, 3 ppm to 2000 ppm, 5 ppm to 1900 ppm, 7 ppm to 1500 ppm, 10 ppm to 1000 ppm, 15 ppm to 800 ppm, 20 ppm to 500 ppm, 25 ppm to 300 ppm, 30 ppm to 200 ppm, 35 ppm to 100 ppm, 40 ppm to 90 ppm, 45 ppm to 70 ppm, or 50 ppm to 70 ppm, based on mass.

The ratio of isocyanatomethylbenzoyl chloride in the XDI composition is measured by the method described in Examples described later.

The XDI composition may further contain isocyanatomethylbenzoic acid as a secondary component.

The ratio of isocyanatomethylbenzoic acid in the XDI composition is, based on mass, for example, 0.1 ppm or more, 0.3 ppm or more, 0.5 ppm or more, 0.7 ppm or more, 1.0 ppm or more, 1.5 ppm or more, 2.0 ppm or more, 2.5 ppm or more, 3.0 ppm or more, 3.5 ppm or more, 4.0 ppm or more, 4.5 ppm or more, and 5.0 ppm or more.

When the ratio of isocyanatomethylbenzoic acid in the XDI composition is the above-described lower limit or more, the heat resistance of the resin produced using the XDI composition can be improved.

The ratio of isocyanatomethylbenzoic acid in the XDI composition is, based on mass, for example, 100.0 ppm or less, 80.0 ppm or less, 50.0 ppm or less, 30.0 ppm or less, 20.0 ppm or less, 10.0 ppm or less, 9.0 ppm or less, or 7.0 ppm or less.

When the ratio of the isocyanatomethylbenzoic acid in the XDI composition is the above-described upper limit or less, the reduction in light resistance of the resin produced using the XDI composition can be suppressed.

The above-described upper and lower limits of the ratio of isocyanatomethylbenzoic acid in the XDI composition can be combined to set a range of the ratio of isocyanatomethylbenzoic acid in the XDI composition. The ratio of isocyanatomethylbenzoic acid in the XDI composition may be, based on mass, 0.1 ppm to 100.0 ppm, 0.3 ppm to 80.0 ppm, 0.5 ppm to 50.0 ppm, 0.7 ppm to 30.0 ppm, 1.0 ppm to 20.0 ppm, 1.5 ppm to 10.0 ppm, 2.0 ppm to 9.0 ppm, 2.5 ppm to 7.0 ppm, 3.0 ppm to 7.0 ppm, 3.5 ppm to 7.0 ppm, 4.0 ppm to 7.0 ppm, 4.5 ppm to 7.0 ppm, or 5.0 ppm to 7.0 ppm.

The ratio of isocyanatomethylbenzoic acid in the XDI composition is measured by gas chromatography mass spectroscopy analysis and gas chromatography analysis under the same measurement conditions as those of the above-described isocyanatomethylbenzoyl chloride.

The XDI composition may further contain, as a secondary component, at least one of monochloromethylbenzyl isocyanate (CBI), dichloromethylbenzyl isocyanate (DCI), dichloromethaneiminomethylbenzyl isocyanate (dichloroimine product) represented by the following chemical formula (1).

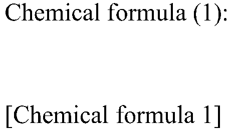

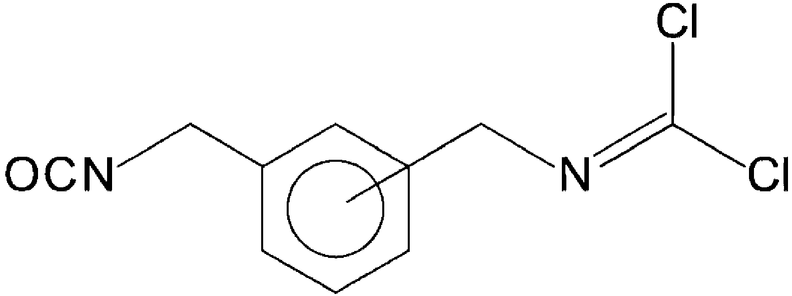

Examples of DCI include 2-(dichloromethyl)benzyl isocyanate (o-DCI), 3-(dichloromethyl)benzyl isocyanate (m-DCI), and 4-(dichloromethyl)benzyl isocyanate (p-DCI).

As DCI, 3-(dichloromethyl)benzilicisocyanate (m-DCI) is preferably used.

The XDI composition may contain two or more types of DCI.

The ratio of DCI in the XDI composition is, based on mass, for example, 0.1 ppm or more, 0.3 ppm or more, 0.6 ppm or more, or 1.0 ppm or more.

The ratio of DCI in the XDI composition is, based on mass, for example, 60 ppm or less, 50 ppm or less, 30 ppm or less, or 20 ppm or less.

The above-described upper and lower limits of the ratio of DCI in the XDI composition can be combined to set a range of the ratio of DCI in the XDI composition. The ratio of DCI in the XDI composition may be, based on mass, 0.1 ppm to 60 ppm, 0.3 ppm to 50 ppm, 0.6 ppm to 30.0 ppm, or 1.0 ppm to 20 ppm.

When the ratio of DCI in the XDI composition falls within the above-described range, the yellowness of the resin produced from the XDI composition can be reduced.

The ratio of DCI in the XDI composition is measured by the method described in Examples below.

Examples of CBI include 2-(monochloromethyl)benzylisocyanate (o-CBI), 3-(monochloromethyl)benzylisocyanate (m-CBI), and 4-(monochloromethyl)benzylisocyanate (p-CBI).

As CBI, 3-(monochloromethyl)benzylisocyanate (m-CBI) is preferably used.

The XDI composition may contain two or more types of CBI.

The ratio of CBI in the XDI composition is, based on mass, for example, 0.2 ppm or more, 6 ppm or more, or 100 ppm or more.

The ratio of CBI in the XDI composition is, based on mass, for example, 5000 ppm or less, 4000 ppm or less, 3000 ppm or less, 1600 ppm or less, or 1000 ppm or less.

The above-described upper and lower limits of the ratio of CBI in the XDI composition can be combined to set a range of the ratio of CBI in the XDI composition. The ratio of CBI in the XDI composition may be, based on mass, 0.2 ppm to 5000 ppm, 6 ppm to 4000 ppm, 100 ppm to 3000 ppm, 100 ppm to 1600 ppm, or 100 ppm to 1000 ppm.

When the ratio of CBI in the XDI composition falls within the above-described range, it is possible to reduce the yellowness of the resin produced from the XDI composition. In particular, when the content ratio of CBI in the XDI composition is the above-described upper limit or less, yellowing of the resin can be suppressed, and the urethanization reaction at the time of producing the resin can be smoothly progressed, and the mechanical properties of the resin can be reliably improved.

In addition, the content ratio of CBI is, for example, 2 times or more, 10 times or more, or 20 times or more, with respect to the content ratio of DCI. The content ratio of CBI is, for example, 800 times or less, 300 times or less, or 50 times or less, with respect to the content ratio of DCI. The content ratio of CBI may be 2 times to 800 times, 10 times to 300 times, or 20 times to 50 times, with respect to the content ratio of DCI.

The ratio of CBI in the XDI composition is measured by the method described in Examples below.

Examples of the dichloroimine product include 2-(dichloromethaneiminomethyl)benzyl isocyanate, 3-(dichloromethaneiminomethyl)benzyl isocyanate, and 4-(dichloromethaneiminomethyl)benzylisocyanate.

As the dichloroimine product, 3-(dichloromethaneiminomethyl)benzylisocyanate is preferably used.

The XDI composition may contain two or more types of dichloroimine products.

The ratio of the dichloroimine product in the XDI composition is, based on mass, for example, 0.1 ppm or more, 0.2 ppm or more, 0.5 ppm or more, 1.0 ppm or more, or 2.0 ppm or more.

The ratio of the dichloroimine product in the XDI composition is, based on mass, for example, 200 ppm or less, 150 ppm or less, 100 ppm or less, 80 ppm or less, or 60 ppm or less.

The above-described upper and lower limits of the ratio of the dichloroimine product in the XDI composition can be combined to set a range of the ratio of the dichloroimine product in the XDI composition. The ratio of the dichloroimine product in the XDI composition may be, based on mass, 0.1 ppm to 200 ppm, 0.2 ppm to 150 ppm, 0.5 ppm to 100 ppm, 1.0 ppm to 80 ppm, or 2.0 to 60 ppm.

The ratio of the dichloroimine product in the XDI composition is measured by the method described in Examples below.

### 2. Method of Producing XDI Composition

A method of producing the XDI composition is described.

The method of producing the XDI composition includes, for example, a synthesis step, a purification step, and a generation step.

In the synthesis step, XDI is synthesized. In the synthesis step, for example, XDI is synthesized by a hydrochloride method. When a hydrochloride method is employed, the synthesis step includes a salt-forming step and an isocyanate-forming step.

In the salt-forming step, xylylenediamine hydrochloric acid (XDA hydrochloride) is produced by mixing xylylenediamine (XDA) and hydrogen chloride.

Examples of XDA include 1,2-XDA (o-XDA), 1,3-XDA (m-XDA), and 1,4-XDA (p-XDA), and preferably 1,3-XDA (m-XDA) is used.

In the salt-forming step, for example, XDA and hydrogen chloride are reacted in the presence of an inert solvent. Specifically, hydrogen chloride gas is mixed with a solution in which XDA is dissolved in the inert solvent to react XDA with hydrogen chloride.

As the inert solvent, for example, the inert solvent described in paragraph [0059] of WO 2018/190290 is used. These inert solvents may be used alone or in combination of two or more. Among the inert solvents, preferably, halogenated aromatic hydrocarbons are used, and more preferably chlorobenzene and dichlorobenzene are used.

The mass ratio (total amine concentration) of XDA to the total sum of the mass of XDA and the inert solvent is, for example, 3% by mass to 30% by mass, 5% by mass to 20% by mass, or 5% by mass to 15% by mass.

The feeding ratio of hydrogen chloride with respect to 1 mol of XDA is, for example, 2 mol to 10 mol, 2 mol to 6 mol, or 2 mol to 4 mol.

The reaction temperature in the salt-forming step is, for example, 30°C to 160°C, 50°C to 150°C, or 50°C to 140°C.

The reaction pressure (gauge pressure) in the salt-forming step is, for example, 0 MPaG (atmospheric pressure) to 1.0MPaG, or 0.01MPaG to 0.5MPaG.

The reaction of XDA with hydrogen chloride generates XDA hydrochloride, thereby producing a slurry that contains XDA hydrochloride.

Next, in the isocyanate-forming step, XDA hydrochloride and the carbonyl dichloride are reacted to produce a reaction mass which contains XDI. In the isocyanate-forming step, carbonyl dichloride is mixed into the slurry containing XDA hydrochloride to react XDA hydrochloride with carbonyl dichloride while removing the hydrogen chloride gas by-produced. The reaction of XDA hydrochloride with carbonyl dichloride generates XDI. That is, XDI is obtained by the reaction of XDA hydrochloride with carbonyl dichloride.

The feeding ratio of carbonyl dichloride with respect to 1 mol of XDA hydrochloride is, for example, 4 mol to 50 mol, 5 mol to 40 mol, or 6 mol to 30 mol.

The reaction time of the isocyanate-forming step is, for example, 4 hours to 25 hours, 6 hours to 20 hours, or 6 hours to 15 hours.

The reaction temperature in the isocyanate-forming step is, for example, 90°C to 190°C, 100°C to 180°C, or 110 to 160°C.

The reaction pressure (gauge pressure) in the isocyanate-forming step is, for example, 0 MPaG to 0.6 MPaG, 0.0005 MPaG to 0.4 MPaG, 0.001 MPaG to 0.2 MPaG, 0.003 MPaG to 0.2 MPaG, 0.01 MPaG to 0.2 MPaG, 0.02 MPaG to 0.2 MPaG, or 0.03 MPaG to 0.2 MPaG. The reaction pressure (gauge pressure) in the isocyanate-forming step is preferably more than 0 MPaG (atmospheric pressure).

The isocyanate-forming step is preferably carried out by a continuous process. In other words, the slurry containing XDA hydrochloride is continuously fed into the reaction tank used in the isocyanate-forming step, and the reaction mass is continuously taken out of the reaction tank while XDA hydrochloride and the carbonyl dichloride are being reacted in the reaction tank.

Next, the gas component, inert solvent, and tar component are removed from the reaction mass.

The gas component contains the carbonyl dichloride remaining in the reaction mass without reacting with XDA hydrochloride in the isocyanate-forming step, and the hydrogen chloride gas by-produced in the isocyanate-forming step. The gas component is removed from the reaction mass, for example, using a known degassing column.

The inert solvent is distilled off from the reaction mass, for example, using a known distillation column.

The tar component is removed from the reaction mass, for example, using a known tar removal device.

After the gas component, inert solvent, and tar component are removed, the ratio of XDI in the reaction mass is, for example, 80.0% by mass to 99.0% by mass, 90.0% by mass to 98.5% by mass, or 95.0% by mass to 98.0% by mass.

Next, in the purification step, the reaction mass is purified. The purification step includes, for example, a low-boiling removing step and a rectification step.

In the low-boiling removing step, a low boiling point component is removed from the reaction mass. The low boiling point component has a boiling point lower than that of XDI. In the low-boiling removing step, for example, the reaction mass is distilled in a low-boiling removal column to distill off the low boiling point component from the reaction mass.

Examples of the low-boiling removal column include a tray column and a packed column, and preferably, a packed column is used. The number of theoretical plates of the low-boiling removal column is, for example, 3 to 40, 5 to 20, or 7 to 15.

The temperature of the bottom of the low-boiling removal column is, for example, 130°C to 200°C, 140°C to 190°C, or 150°C to 180°C.

The temperature of the top of the low-boiling removal column is, for example, 90°C to 160°C, 100°C to 150°C, or 110°C to 140°C.

The pressure of the top of the low-boiling removal column is, for example, 0.05 kPa to 3.0 kPa, 0.1 kPa to 2.0 kPa, or 0.2 kPa to 1.0 kPa.

The reflux ratio of the top of the low-boiling removal column is, for example, 1 to 80, 5 to 60, or 10 to 50.

The residence time of the low-boiling removal column is, for example, 0.1 hours to 10 hours, 0.2 hours to 5 hours, or 0.3 hours to 3 hours.

By distillation using the low-boiling removal column, the reaction mass from which a low boiling point component is distilled off is obtained as a can effluent.

Next, in the rectification step, the reaction mass after the low-boiling removing step is further distilled (rectified) using a rectification column.

Examples of the rectification column include a tray column and a packed column, and preferably, a packed column is used. The number of theoretical plates of the rectification column is, for example, 1 to 20, 1 to 10, or 1 to 5.

The temperature of the bottom of the rectification column is, for example, 120°C to 190°C, 130°C to 180°C, or 140°C to 170°C.

The temperature of the top of the rectification column is, for example, 90 to 180°C, 110 to 170°C, or 130 to 160°C.

The pressure of the top of the rectification column is, for example, 0.05 kPa to 3.0 kPa, 0.1 kPa to 2.0 kPa, or 0.2 kPa to 1.0 kPa.

The reflux ratio of the top of the rectification column is, for example, 0.1 to 50, 0.2 to 20, or 0.3 to 10.

The residence time of the rectification column is, for example, 0.2 hours to 20 hours, 0.5 hours to 10 hours, or 1.0 hours to 10 hours.

By the rectification step, a fraction (first XDI composition) that contains XDI is produced. The first XDI composition contains DCI, CBI, and a dichloroimine product.

Next, in the generation step, isocyanatomethylbenzoic acid and isocyanatomethylbenzoyl chloride are generated.

In detail, in the generation step, compressed air is injected into the first XDI composition.

The temperature of the first XDI composition in the generation step is, for example, 10°C to 80°C, 15 to 70°C, 20°C to 50°C, or 20°C to 30°C.

The flow rate of the compressed air is, for example, 10ml/min to 30ml/min, or 15ml/min to 25ml/min, with respect to 100g of the first XDI composition.

The time for injecting the compressed air is, for example, 10 minutes to 20 hours, 1 hour to 15 hours, 2 hours to 10 hours, and 5 hours to 10 hours.

By the generation step, isocyanatomethylbenzoic acid and isocyanatomethylbenzoyl chloride are generated. In detail, it is considered that injecting the compressed air into the first XDI composition causes the reaction between the by-product in the first XDI composition and oxygen, thereby generating isocyanatomethylbenzoic acid. Further, it is considered that the resulting isocyanatomethylbenzoic acid is allowed to react with the hydrolyzable chlorine in the first XDI composition, thereby generating isocyanatomethylbenzoyl chloride.

By the generation step, the above-described XDI composition (second XDI composition) is obtained.

In addition, the method of producing the XDI composition does not include the generation step, and may include, for example, the salt-forming step, the isocyanate-forming step, the purification step, and the mixing step.

In the mixing step, isocyanatomethylbenzoic acid or isocyanatomethylbenzoyl chloride is added to the first XDI composition, and mixed.

Also in this method, the above-described XDI composition can be obtained.

### 3. Uses of the XDI composition

The above-described XDI composition is utilized as a raw material for a resin. The resin is produced by reacting an isocyanate component containing the XDI composition with an active hydrogen group-containing component containing an active hydrogen group-containing compound.

The active hydrogen group-containing compound contains an active hydrogen group. The active hydrogen group is a functional group capable of generating active hydrogen. Examples of the active hydrogen group include a hydroxy group, a mercapto group, and an amino group. Examples of the active hydrogen group-containing compound include a polyol, a polythiol, and a polyamine.

The active hydrogen group-containing compound can be used alone or in combination of two or more.

The active hydrogen group-containing compound is preferably a polythiol from the viewpoint of optical characteristics. The active hydrogen group-containing component is preferably a polythiol composition containing a polythiol as a primary component.

The ratio of polythiol in the polythiol composition is, for example, 50% by mass or more, 60% by mass or more, 70% by mass or more, or 80% by mass or more.

The ratio of polythiol in the polythiol composition is measured, for example, by high performance liquid chromatography.

The polythiol has a plurality of mercapto groups. The polythiol does not contain a secondary component to be described later. Examples of the polythiol include an aliphatic polythiol, an aromatic polythiol, and a heterocyclic polythiol.

Examples of the aliphatic polythiol include methanedithiol, 1,2-ethanedithiol, 1,2,3-propanetritiol, 1,2-cyclohexanedithiol, bis(2-mercaptoethyl)ether, tetrakis(mercaptomethyl)methane, diethylene glycol bis(2-mercaptoacetate), diethylene glycol bis(3-mercaptopropionate), ethylene glycol bis(2- mercaptoacetate), ethylene glycol bis(3-mercaptopropionate), trimethylolpropanetris(2-mercaptoacetate), trimethylolpropanetris(3-captopropionate), trimethylolethantris (2-mercaptoacetate), trimethylolethantris (3-mercaptopropionate), pentaerythritol tetrakis (2-mercaptoacetate), pentaerythritol tetrakis(3-mercaptopropionate), bis(mercaptomethyl)sulfide, bis(mercaptomethyl)disulfide, bis(mercaptoethyl) sulfide, bis(mercaptoethyl)disulfide, bis(mercaptopropyl)sulfide, bis(mercaptomethylthio)methane, bis(2-mercaptoethylthio)methane, bis(3-mercaptopropylthio)methane, 1,2-bis(mercaptomethylthio)ethane, 1,2-bis(2-mercaptoethylthio)ethane, 1,2-bis(3-mercaptopropylthio)ethane, 1,2,3-tris(mercaptomethylthio)propane, 1,2,3-tris(2-mercaptoethylthio)propane, 1,2,3-tris(3-mercaptopropylthio)propane, 4-mercaptomethyl-1,8-dimercapto-3,6-dithiaoctane, 5,7-dimercaptomethyl-1,11-dimercapto-3,6,9-trithiaundecane, 4,7-dimercaptomethyl-1,11-dimercapto-3,6,9-trithiaundecane, 4,8-dimercaptomethyl-1,11-dimercapto-3,6,9-trithiaundecane, tetrakis(mercaptomethylthiomethyl)methane, tetrakis(2-mercaptoethylthiomethyl)methane, tetrakis(3-mercaptopropylthiomethyl)methane, bis(2,3-dimercaptopropyl) sulfide, 2,5-dimercaptomethyl-1,4-dithiane, 2,5-dimercapto-1,4-dithiane, 2,5-dimercaptomethyl-2,5-dimethyl-1,4-dithiane, and esters of thioglycolic acids and mercaptopropionic acids thereof, hydroxymethyl sulfide bis (2-mercaptoacetate), hydroxymethyl sulfide bis (3-mercaptopropionate), hydroxyethyl sulfide bis(2-mercaptoacetate), hydroxyethyl sulfide bis(3-mercaptopropionate), hydroxymethyl disulfide bis(2-mercaptoacetate), hydroxymethyl disulfide bis(3-mercaptopropionate), hydroxyethyl disulfide bis(2-mercaptoacetate), hydroxyethyl disulfide bis(3-mercaptopropionate), thiodiglycolic acid bis(2-mercaptoethyl ester), thiodipropionic acid bis(2-mercaptoethyl ester), dithiodiglycolic acid bis(2-mercaptoethyl ester), dithiodipropionic acid bis(2-mercaptoethyl ester), 1,1,3,3-tetrakis(mercaptomethylthio)propane, 1,1,2,2-tetrakis(mercaptomethylthio)ethane, 4,6-bis(mercaptomethylthio)-1,3-dithiane, 2-(2,2-bis(mercaptomethylthio)ethyl)-1,3-dithiethane, 3-mercaptomethyl-1,5-dimercapto-2,4-dithiapentane, tris(mercaptomethylthio)methane, and tris(mercaptoethylthio)methane.

Examples of the aromatic polythiol include 1,2-dimercaptobenzene, 1,3-dimercaptobenzene, 1,4-dimercaptobenzene, 1,2-bis(mercaptomethyl)benzene, 1,3-bis(mercaptomethyl)benzene, 1,4-bis(mercaptomethyl)benzene, 1,2-bis(mercaptoethyl)benzene, 1,3-bis(mercaptoethyl)benzene, 1,4-bis(mercaptoethyl)benzene, 1,3,5-trimercaptobenzene, 1,3,5-tris(mercaptomethyl)benzene, 1,3,5-tris(mercaptomethyleneoxy)benzene, 1,3,5-tris(mercaptoethyleneoxy)benzene, 2,5-toluenedithiol, 3,4-toluenedithiol, 1,5-naphthalenedithiol, and 2,6-naphthalenedithiol.

Examples of the heterocyclic polythiol include 2-methylamino-4,6-dithiol-sym-triazine, 3,4-thiophenedithiol, and bismuthiol.

The polythiols may be used alone or in combination of two or more.

Preferably, examples of the polythiol include at least one type selected from the group consisting of 5,7-dimercaptomethyl-1,11-dimercapto-3,6,9-trithiaundecane, 4,7-dimercaptomethyl-1,11-dimercapto-3,6,9-trithiaundecane, 4,8-dimercaptomethyl-1,11-dimercapto-3,6,9-trithiaundecane, 4-mercaptomethyl-1,8-dimercapto-3,6-dithiaoctane, pentaerythritol tetrakis(2-mercaptoacetate), pentaerythritol tetrakis(3-mercaptopropionate), 2,5-bis(mercaptomethyl)-1,4-dithiane, bis(mercaptoethyl)sulfide, 1,1,3,3-tetrakis(mercaptomethylthio)propane, 4,6-bis(mercaptomethylthio)-1,3-dithiane, 2-(2,2-bis(mercaptomethylthio)ethyl)-1,3-dithiethane, 1,1,2,2-tetrakis(mercaptomethylthio)ethane, 3-mercaptomethyl-1,5-dimercapto-2,4-dithiapentane, tris(mercaptomethylthio)methane, ethylene glycol bis(3-mercaptopropionate), and diethylene glycol bis(3-mercaptopropionate).

The polythiol composition may contain a secondary component.

Examples of the secondary component include a compound in which at least one of a plurality of mercapto groups of the above-described polythiol is substituted with a functional group represented by the following chemical formula (2) (hereinafter, referred to as a compound A).

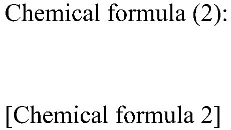

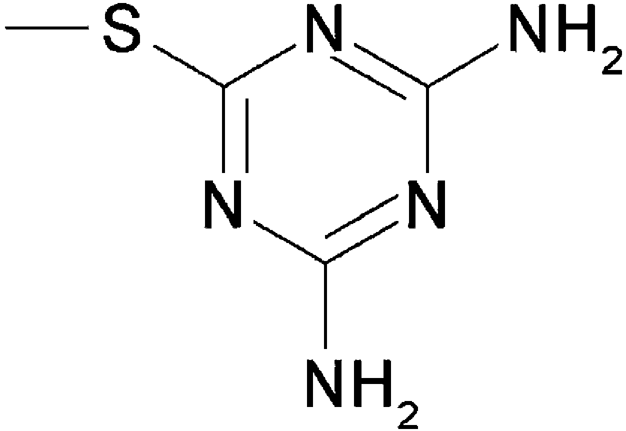

When the polythiol composition contains the compound A, in high performance liquid chromatography measurement of the polythiol composition, the peak area (R1) of the compound A with respect to the peak area 100 of the polythiol is, for example, 0.01 to 3.0, 0.01 to 1.5, or 0.01 to 0.5.

The "peak area (R1) of the compound A with respect to the peak area 100 of the polythiol" means the relative value (ratio) of the peak area (P_{A}) of the compound A when the peak area of the polythiol (Pₜₕᵢₒₗ) (including structural isomers of the polythiol) (Pₜₕᵢₒₗ) is 100, and is calculated according to the following formula (1).$R 1 = \left({P}_{A}/{P}_{thiol}\right) \times 100$

When the "peak area (R1) of the compound A with respect to the peak area 100 of the polythiol" is determined, the high performance liquid chromatography measurement is carried out under the measurement conditions described in paragraph [0041] of WO2022/102625.

When the peak area (R1) of the compound A with respect to the peak area 100 of the polythiol falls within the above-described range, the pot life of the polymerizable composition obtained from the polythiol composition and the polyisocyanate composition can be maintained sufficiently, and further, by curing the polymerizable composition, a plastic lens made of a polythio urethane resin having excellent qualities, such as hue, transparency, striae, can be obtained.

In addition, when the polythiol composition contains at least one type of polythiol selected from the group consisting of 4-mercaptomethyl-1,8-dimercapto-3,6-dithiaoctane, 5,7-dimercaptomethyl-1,11-dimercapto-3,6,9-trithiaundecane, 4,7-dimercaptomethyl-1,11-dimercapto-3,6,9-trithiaundecane, and 4,8-dimercaptomethyl-1,11-dimercapto-3,6,9-trithiaundecane, the polythiol composition may further contain a compound represented by the following chemical formula (3) (hereinafter, referred to as a compound B) as a secondary component. 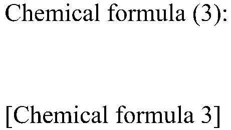 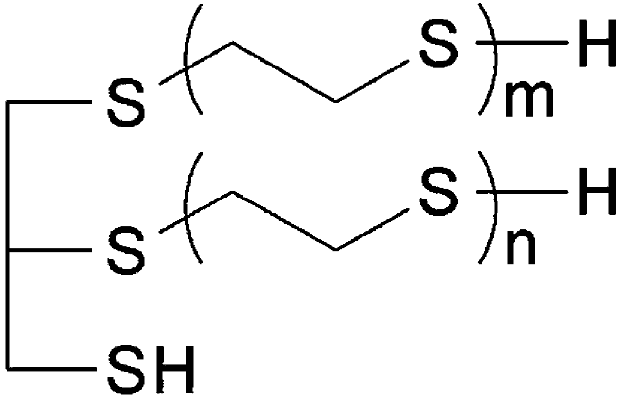 (In Formula (3), each of m and n independently represents 0 or 1, and m+n=1 is satisfied.)

When the polythiol composition contains the compound B, in the high performance liquid chromatography measurement of the polythiol composition, the peak area (R2) of the compound B with respect to the total peak area 100 of the compound contained in the polythiol composition is, for example, more than 0 and 10.0 or less, 0.02 to 9.0, 0.04 to 8.0, 1.0 to 7.0, 2.0 to 6.0, 3.0 to 6.0, or 4.0 to 6.0.

The "peak area (R2) of the compound B with respect to the total peak area 100 of the compound contained in the polythiol composition" means the relative value (ratio) of the peak area (P_{B}) of the compound B when the total peak area (Pₛᵤₘ) of the compound contained in the polythiol composition is 100, and is calculated according to the following formula (2). The "total peak area (Pₛᵤₘ) of the compound contained in the polythiol composition" is the total sum of the peak area of all the peaks detected in the high-performance liquid chromatography measurement of the polythiol composition. $R 2 = \left({P}_{B}/{P}_{sum}\right) \times 100$

When the "peak area (R2) of the compound B with respect to the total peak area 100 of the compound contained in the polythiol composition" is determined, the high performance liquid chromatography measurement is carried out under the measurement conditions described in paragraph [0049] of WO2022/138865.

When the peak area (R2) of the compound B with respect to the total peak area 100 of the compound contained in the polythiol composition is the above-described upper limit or less, it is possible to improve the light resistance of the resin produced from the polythiol composition. Further, when the peak area (R2) of the compound B with respect to the total peak area 100 of the compound contained in the polythiol composition is the above-described lower limit or more, it is possible to improve the dyeability of the resin produced from the polythiol composition.

For example, the resin is manufactured by casting molding. In the casting molding, first, the isocyanate component and the active hydrogen group-containing component are mixed in a ratio in which the isocyanate group in the isocyanate component to the active hydrogen group (amino group, thiol group, or hydroxyl group) in the active hydrogen group-containing component is 0.8 to 1.2. The resulting mixture is the polymerizable composition containing the XDI composition and the active hydrogen group-containing component.

In addition, a known additive may be mixed into the polymerizable composition. Examples of the additive include a curing catalyst, a stabilizer (acidic phosphate ester), and an ultraviolet absorber.

Next, the polymerizable composition is injected into a mold, and then heated and cured. Thus, a molded article made of the resin is obtained. In other words, the resin is a cured product of the polymerizable composition.

When the active hydrogen group-containing component contains the polythiol, the obtained molded article is excellent in transparency.

Furthermore, the above-described XDI composition contains XDI and isocyanatomethylbenzoyl chloride, and thus the obtained molded article has low yellowness and excellent heat resistance.

Specifically, the obtained molded article has a glass transition temperature (Tg) of, for example, 85.0°C or more, 87.0°C or more, or 87.5°C or more. The glass transition temperature (Tg) of the obtained molded article is, for example, 90.0°C or less, or 88.0°C or less. The glass-transition temperature (Tg) of the obtained molded article may be 85.0°C to 90.0°C, 87.0°C to 88.0°C, or 87.5°C to 88.0°C.

The obtained molded article has a YI value of, for example, 5.85 or less, 5.65 or less, 5.60 or less, 5.55 or less, 5.50 or less, 5.45 or less, or 5.43 or less. The YI value of the obtained molded article is, for example, 5.40 or more. The YI value of the obtained molded article may be 5.40 to 5.85, 5.40 to 5.65, 5.40 to 5.55, 5.40 to 5.50, 5.40 to 5.45, or 5.40 to 5.43.

The obtained molded article also has a high refractive index. The obtained molded article has a refractive index (ne) of, for example, 1.650 or more, or 1.660 or more. The refractive index (ne) of the obtained molded article is, for example, 1.670 or less. The refractive index (ne) of the obtained molded article may be 1.650 to 1.670, or 1.660 to 1.670.

The Abbe number (νe) of the obtained molded article is, for example, 30 or more, or 31 or more. The Abbe number (νe) of the obtained molded article is, for example, 35 or less, or 33 or less. The Abbe number (νe) of the obtained molded article may be 30 to 35 or 31 to 33.

When having the above-described physical properties, the obtained molded article is suitable as an optical element.

Examples of the optical element include a lens, a sheet, and a film, and preferably a lens is used.

Examples of the lens include, for example, a transparent lens, a sunglasses lens, a polarizing lens, an eyeglass lens, a camera lens, a pickup lens, and a contact lens.

The uses of the XDI composition are not limited to the above-described optical materials. The uses of the XDI composition include inks, transfer foils, pressure-sensitive adhesives, binders, gels, elastomers, foams, adhesives, one-component curing sealants, RIM moldings, microcell polyurethanes, various microcapsules, aqueous resins, thermosetting resins, active energy rays (e.g., electron beam, ultraviolet light, etc.) curable resins, artificial and synthetic leathers, slush powders, robotic members, mobility members, health care materials, substrate resins of carbon-fiber reinforced plastics (CFRPs), transparent rubber, transparent hard resins, waterproof materials, films, sheets, tubes, blades, loudspeakers, sensors, organic EL members, photovoltaic members, android members, wearable members, sports items, leisure items, medical products, nursing care products, house components, audio components, lighting components, chandeliers, outdoor wall lights, packings, vibration-proof, vibration-control, and seismic-isolated members, acoustic insulation components, daily use items, sundry items, cushions, bedclothes, stress absorbers, stress relaxation materials, car interior or exterior materials, components for transportation, components for office automation equipment, sundry item-surface protection materials, self-repairing materials, and health appliances.

The uses of the XDI composition preferably include the above-described optical materials, elastomers, foams, and one-component curing sealants.

### 4. Operations and Effects

The xylylene diisocyanate composition and polymerizable composition of the present invention contain isocyanatomethylbenzoyl chloride.

By using the xylylene diisocyanate composition and polymerizable composition, both of which contain isocyanatomethylbenzoyl chloride, as a raw material, a resin having excellent light resistance and heat resistance can be produced.

The resin of the present invention is a cured product of the polymerizable composition containing the above-described xylylene diisocyanate composition.

Further, the molded article, optical element, and lens of the present invention are made of the resin.

Therefore, the resin, molded article, optical element, and lens of the present invention have excellent light resistance and heat resistance.

### 5. Modified Examples

(1) The method of synthesis of XDI is not limited to the above-described hydrochloride method. Examples of the method of synthesis of XDI include a gas phase method in which vaporized XDA is reacted with a carbonyl dichloride, a single step method in which XDA and a carbonyl dichloride are directly reacted in one step, and a cold and hot two-step method in which XDA and a carbonyl dichloride are reacted at a low temperature and then subsequently reacted at a high temperature. In these methods, XDI is obtained by reacting XDA with a carbonyl dichloride. Further, examples of a method of synthesis of XDI include a non-phosgene method in which a xylylene dicarbamate is thermally decomposed to obtain XDI.
(2) The production of the XDI composition may not be carried out continuously in the same plant. For example, the first XDI composition may be produced in the first plant and then used to carry out the generation step in the second plant.

### Examples

Next, the present invention is described in reference to Examples below. The present invention is however not limited by the following Examples. The specific numeral values used in the description below, such as mixing ratios (content ratios), physical property values, and parameters, can be replaced with the corresponding mixing ratios (content ratios), physical property values, and parameters in the above-described "DESCRIPTION OF THE EMBODIMENTS", including the upper limit values (numeral values defined with "or less", and "less than") or the lower limit values (numeral values defined with "or more", and "more than"). The "ratio" of each component is based on mass.

### 1. Production of XDI composition

### (1) Example 1

136g (1.0 mol) of m-xylylenediamine (manufactured by FUJIFILM Wako Pure Chemical Corporation) and 621g of o-dichlorobenzene (ODCB) were mixed in a conical flask to prepare a dropwise solution.

Next, 845g of ODCB was charged into a 1L flask equipped with a reflux condenser, a thermometer, and a gas-injection tube.

The temperature of the ODCB was raised to 100°C, while the ODCB was being stirred, the above-described dropwise solution was added dropwise at a flow rate of 2mL per minute by using a feed pump (HPLC pump PU-980 manufactured by JASCO Corporation) for 3 hours. Meanwhile, at the same time as the dropwise of the dropwise solution, 230g of hydrochloric acid gas was injected for 3 hours.

Next, the injection of hydrochloric acid gas was stopped, and the mixture was heated and stirred for 30 minutes. Thus, a slurry of m-xylylenediamine hydrochloride was obtained (salt-forming step).

Next, nitrogen gas was injected into the slurry for 1 hour to remove unreacted hydrochloric acid gas from the slurry.

Next, the temperature of the slurry was raised to 160°C, and 1200g of carbonyl dichloride gas was injected into the slurry for 6 hours. In this manner, m-xylylenediamine hydrochloride and carbonyl dichloride were reacted to obtain a reaction mass containing m-XDI

### (isocyanate-forming step)

Next, nitrogen gas was injected into the reaction mass for 1 hour to remove unreacted carbonyl dichloride from the reaction mass.

Next, the reaction mass was filtered under reduced pressure to remove the precipitate in the reaction mass.

Next, the reaction mass after the removal of the precipitate was desolvated to distill ODCB in the reaction mass (desolvation step)

Next, using a rectifying column with a filling material corresponding to the four plates, the reaction mass was distilled under reduced pressure (a degree of reduced pressure 0.5 to 1.0 Torr), and from the reaction mass, 158g of a first XDI composition containing m-XDI was obtained as a fraction (rectification step).

Next, 100g of the first XDI composition was charged into a 200ml eggplant flask, and compressed air was injected into the first XDI composition (temperature of the first XDI composition: 25°C, flow rate of the compressed air: 20ml/min, injection time: 20 min) (generation step)

Thereafter, the first XDI composition was filtered with a 1µm Teflon (registered trademark) filter, and 75mg of phenol (stabilizer) was added to prepare a XDI composition (second XDI composition).

### (2) Example 2

A XDI composition was prepared in the same manner as in Example 1 except that compressed air was injected for 2 hours in the generation step.

### (3) Example 3

A XDI composition was prepared in the same manner as in Example 1 except that compressed air was injected for 6 hours in the generation step.

### (4) Example 4

A XDI composition was prepared in the same manner as in Example 1 except that compressed air was injected for 15 hours in the generation step.

### (5) Example 5

A XDI composition was prepared in the same manner as in Example 1 except that the temperature of the first XDI composition was raised to 60°C, and compressed air was injected for 5 hours in the generation step.

### (6) Example 6

A XDI composition was prepared in the same manner as in Example 1 except that the temperature of the first XDI composition was raised to 60°C, and compressed air was injected for 10 hours in the generation step.

### (7) Comparative Example

A XDI composition was prepared in the same manner as in Example 1 except that hydrochloric acid gas equivalent to 20 ppm was injected in place of compressed air in the generation step.

### 2. Quantitative Measurement of XDI Composition

The ratios of XDI, isocyanatomethylbenzoyl chloride (acid chloride), dichloroimine product, DCI, and CBI in the XDI composition obtained in each of Examples and Comparative Examples were measured by the following method. The results are shown in Table 1.

### (1) Identification of Isocyanatomethylbenzoyl Chloride

The XDI composition obtained in Example 1 was subjected to gas chromatography mass spectroscopy analysis under the following conditions:

### (Measurement Conditions)

Equipment; Agillent 6890/5973N
Column; HP19091L-433 HP-50 + (inner diameter 0.25mm× length 30m, film 0.25 µm)
Oven temperature; maintained at 50°C for 1 minute, rising from 50°C to 280°C at 10°C/min, and maintained for 10 minutes after reaching 280°C
Carrier gas; contact flow mode, He, 1.0mL/min
Injection method; Pulsed Splitless method (150 kPa, at 0.5 min)
Injection volume; 1.0µL
Sample concentration; 1.0% mass dichloromethane solution
Injection temperature; 200°C
Interface temperature; 280°C
Quadrupole temperature; 150°C
Ion source temperature; 230°C
Detection method; Scan method (m/z: 10 to 500)

The mass spectrum of the peak which appeared at the retention time of 19.87 minutes in the obtained total ion chromatogram was compared with library data.

The mass spectrum included a fragment peak with a molecular weight of 195 as the maximum peak, and had a degree of agreement of 70% or more with isocyanatomethylbenzoyl chloride.

Therefore, the compound corresponding to the peak which appeared at the retention time of 19.87 minutes in the total ion chromatogram was identified as isocyanatomethylbenzoyl chloride.

### (2) Ratios of XDI, Isocyanatomethylbenzoyl Chloride, and Dichloroimine Product in XDI Composition

100 mg of the XDI composition and 100 mg of 1,2,4,5-tetrachlorobenzene as an internal standard material were mixed together. Next, the obtained mixture was diluted with dichloromethane so that the volume was 10 mL, thereby obtaining a sample. Next, the obtained sample was subjected to a gas chromatography analysis under the following measurement conditions.

### (Measurement Conditions)

Equipment; SHIMADZU 2014 (manufactured by Shimadzu Corporation)
Packing material; DB-1 (film thickness) 1.5 µm,
Column; internal diameter 0.53 mm × length 60 m (manufactured by Shimadzu Corporation)
Oven temperature; rising from 130°C to 220°C at 3°C/min, after reaching 220°C, rising to 300°C at 10°C/min.
Split ratio; pulsed splitless method
Injection port temperature; 280°C
Detector temperature; 300°C
Carrier gas; N₂ 158 kPa, H₂ 55 kPa, Air 45 kPa (Constant pressure control)
Injection volume; 2 µL
Detection Method; FID

The ratio of XDI in the XDI composition was calculated from the area ratio based on the peak of the internal standard material which appeared at the retention time of 8.8 minutes and the peak of the XDI which appeared at the retention time of 13.8 minutes.

In addition, the ratio of isocyanatomethylbenzoyl chloride in the XDI composition was calculated from the area ratio based on the peak of the internal standard material and the peak of isocyanatomethylbenzoyl chloride which appeared at the retention time of 17.8 minutes.

In addition, the ratio of the dichloroimine product in the XDI composition was calculated from the area ratio based on the peak of the internal standard material and the peak of the dichloroimine product which appeared at the retention time of 15.6 minutes.

### (3) Ratio of DCI in XDI Composition

The ratio of DCI in the XDI composition was measured by the method described in paragraphs [0375] and [0376] of WO2018/190290.

### (4) Ratio of CBI in XDI Composition

The ratio of CBI in the XDI composition was measured by the method described in paragraphs [0376] and [0377] of WO2018/190290.

### [Table 1]

**Table 1**

| | | | Example 1 | Example 2 | Example 3 | Example 4 | Example 5 | Example 6 | Comp. Ex. 1 |
|---|---|---|---|---|---|---|---|---|---|
| Compositions | XDI (% by mass) | | 99.8 | 99.7 | 99.7 | 99.6 | 99.5 | 99.5 | 99.8 |
| | Acid chloride (ppm) | | 10.0 | 50.0 | 100.0 | 300.0 | 800.0 | 2000.0 | 0.0 |
| | Dichloroimine product (ppm) | | 5.0 | 5.0 | 5.0 | 5.0 | 5.0 | 5.0 | 5.0 |
| | CBI (ppm) | | 1000.0 | 1000.0 | 1000.0 | 1000.0 | 1000.0 | 1000.0 | 1000.0 |
| | DCI (ppm) | | 0.8 | 0.8 | 1.0 | 1.0 | 1.0 | 1.0 | 0.6 |
| Resin properties | YT value | | 5.40 | 5.41 | 5.45 | 5.50 | 5.61 | 5.87 | 5.39 |
| | Tg | | 87.5 | 87.6 | 87.8 | 87.7 | 88.4 | 88.5 | 87.1 |
| | Light resistance | 0 hr | 2.29 | 2.34 | 2.32 | 2.35 | 2.38 | 2.40 | 2.30 |
| | | 150hr | 3.85 | 3.90 | 3.95 | 4.01 | 4.10 | 4.30 | 3.80 |
| | | ΔYI | 1.56 | 1.56 | 1.63 | 1.66 | 1.72 | 1.90 | 1.50 |

### 2. Production of Molded Article

0.01 parts by mass of dimethyltin dichloride as a curing catalyst, 0.10 parts by mass of ZELEC UN (trade name manufactured by Stepan Company; acidic phosphate ester), and 1.5 parts by mass of Biosorb 583 (manufactured by SAKAI CHEMICAL INDUSTRY CO.,LTD.; ultraviolet absorber) were mixed into 52 parts by mass of the XDI composition shown in Table 1 at 20°C, and dissolved to obtain a mixed solution 1.

Next, 48 parts by mass of a 4,8-dimercaptomethyl-1,8-dimercapto-3,6-dithiaoctane (manufactured by Mitsui Chemicals, Inc.) was homogenously mixed into the mixed solution 1 to obtain a mixed solution 2 (polymerizable composition).

The mixed solution 2 was defoamed at 600Pa for 1 hour, and then filtered through a 1µm Teflon (registered trademark) filter.

Next, the filtered mixed solution 2 was injected into a mold formed of a glass mold and a tape.

Next, the mold into which the mixed solution 2 was injected was put into an oven, and the temperature was raised from 10°C to 120°C, and the mixed solution 2 was cured at 120°C for 38 hours.

Thereafter, the mold was removed from the oven, and the cured product was released from the mold, and the obtained cured product was annealed at 120°C for 1 hour.

As above-described, the cured product (molded article) of each of Examples and Comparative Examples was obtained.

### 3. Evaluations of Physical Properties of Molded Articles

### (1) Light Resistance

A flat plate with a thick of 2 mm was produced from the obtained molded article, and a QUV test (light source: UVA-340, strength: 0.50W/m², test conditions: 50°C × 150 hours) was carried out using a weather meter manufactured by Q-Lab.

The yellowness (YI value) of the molded article before and after the QUV test was measured, and the difference between the yellowness of the molded article before the QUV test and the yellowness of the molded article after the QUV test (the amount of change in yellowness, ΔYI) was calculated. The smaller the ΔYI is, the better the light resistance is.

The ΔYI value of each of Examples and Comparative Examples is shown in Table 1.

### (2) Yellowness (YI Value)

A circular flat plate with a thickness 9mm and a diameter 75mm was produced from the obtained resins, and the YI value was obtained using a spectrophotometric colorimeter CM-5 manufactured by KONICA MINOLTA JAPAN, INC.

The smaller the YI value is, the lower the yellowness of the resin is, and the larger the YI value is, the higher the yellowness of the resin is.

The YI value of each of Examples and Comparative Examples is shown in Table 1.

### (3) Heat Resistance

A test piece of length 10 mm, width 10 mm, and thickness 2.5 mm was produced from the obtained resin, and the glass-transition temperature (Tg) was measured by a TMA penetration method (50 g load, pin end 0.5 mmφ, temperature increase rate 10°C/min) using a thermomechanical analyzer TMA-60 manufactured by Shimadzu Corporation. The higher the glass-transition temperature (Tg) is, the better the heat resistance is.

The glass-transition temperature (Tg) of each of Examples and Comparative Examples is shown in Table 1.

### (1) Refractive index (ne) and Abbe number (νe)

A test piece of length 10mm, width 10mm, and thickness 2.5mm was produced from the obtained resin, and the refractive index (ne) at a wave length 546.1 nm (mercury e-line), the refractive index (nF') at a wave length 480.0nm (Cd F' line), and the refractive index (nC') at a wave length 643.9 nm (Cd C' line) were each measured by using a Pulfrich refractometer KPR-30 manufactured by Shimadzu Corporation. The Abbe number (νe) was obtained based on the refractive index (ne), the refractive index (nF'), and the refractive index (nC').

In all of Examples and Comparative Examples, the refractive index (ne) was 1.665 and the Abbe number (νe) was 31.

While the illustrative embodiments of the present invention are provided in the above-described description, such is for illustrative purpose only and it is not to be construed as limiting in any manner. Modification and variation of the present invention that will be obvious to those skilled in the art is to be covered by the following claims.

### Industrial Applicability

The xylylene diisocyanate composition, polymerizable composition, resin, and molded article of the present invention can be used, for example, for producing an optical element such as a lens.

## Claims

1. A xylylene diisocyanate composition comprising:
a xylylene diisocyanate; and
isocyanatomethylbenzoyl chloride.

2. The xylylene diisocyanate composition according to claim 1, wherein a ratio of the isocyanatomethylbenzoyl chloride in the xylylene diisocyanate composition is 1 ppm or more based on mass.

3. The xylylene diisocyanate composition according to claim 1, wherein a ratio of the isocyanatomethylbenzoyl chloride in the xylylene diisocyanate composition is 2000 ppm or less based on mass.

4. The xylylene diisocyanate composition according to claim 1, further comprising: isocyanatomethylbenzoic acid.

5. A polymerizable composition comprising:
the xylylene diisocyanate composition according to any one of claims 1 to 4; and
an active hydrogen group-containing component.

6. The polymerizable composition according to claim 5, wherein the active hydrogen group-containing component contains at least one type of polythiol selected from the group consisting of 5,7-dimercaptomethyl-1,11-dimercapto-3,6,9-trithiaundecan, 4,7-dimercaptomethyl-1,11-dimercapto-3,6,9-trithiaundecan, 4,8-dimercaptomethyl-1,11-dimercapto-3,6,9-trithiaundecan, 4-mercaptomethyl-1,8-dimercapto-3,6-dithiaoctane, pentaerythritol tetrakis(2-mercaptoacetate), pentaerythritol tetrakis(3-mercaptopropionate), 2,5-bis(mercaptomethyl)-1,4-dithiane, bis(mercaptoethyl)sulfide, 1,1,3,3-tetrakis(mercaptomethylthio)propane, 4,6-bis(mercaptomethylthio)-1,3-dithiane, 2-(2,2-bis(mercaptomethylthio)ethyl)-1,3-dithiethane, 1,1,2,2-tetrakis(mercaptomethylthio)ethane, 3-mercaptomethyl-1,5-dimercapto-2,4-dithiapentane, tris(mercaptomethylthio)methane, and ethylene glycol bis(3-mercaptopropionate).

7. A resin being a cured product of the polymerizable composition according to claim 5.

8. A molded article made of the resin according to claim 7.

9. An optical element being the molded article according to claim 8.

10. A lens being the optical element according to claim 9.
